(19) Europäisches Patentamt
European Patent Office
Office européen des brevets

(11) **EP 2 062 599 A1**

(12) **EUROPEAN PATENT APPLICATION**
published in accordance with Art. 153(4) EPC

(43) Date of publication:
**27.05.2009 Bulletin 2009/22**

(21) Application number: **07807212.1**

(22) Date of filing: **13.09.2007**

(51) Int Cl.:
*A61K 47/36* (2006.01)    *A61K 9/20* (2006.01)
*A61K 47/10* (2006.01)    *A61K 47/26* (2006.01)
*A61K 47/38* (2006.01)

(86) International application number:
**PCT/JP2007/067805**

(87) International publication number:
**WO 2008/032767 (20.03.2008 Gazette 2008/12)**

(84) Designated Contracting States:
**AT BE BG CH CY CZ DE DK EE ES FI FR GB GR HU IE IS IT LI LT LU LV MC MT NL PL PT RO SE SI SK TR**
Designated Extension States:
**AL BA HR MK RS**

(30) Priority: **14.09.2006 US 844396 P**

(71) Applicant: **Astellas Pharma Inc.**
**Tokyo 103-8411 (JP)**

(72) Inventors:
• **TSUSHIMA, Yuki**
  **Tokyo 103-8411 (JP)**
• **NAKAMURA, Soichiro**
  **Tokyo 103-8411 (JP)**

(74) Representative: **HOFFMANN EITLE**
**Patent- und Rechtsanwälte**
**Arabellastrasse 4**
**81925 München (DE)**

(54) **ORALLY DISINTEGRATING TABLET AND PROCESS FOR PRODUCTION THEREOF**

(57) A rapidly disintegrating tablet in the buccal cavity, comprising a drug, a treated starch having a degree of gelatinization of 30% to 60%, and a saccharide, and a process for manufacturing the same comprising the steps of mixing a drug, a treated starch having a degree of gelatinization of 30% to 60%, and a saccharide, and tabletting the mixture, are disclosed. The rapidly disintegrating tablet in the buccal cavity has a rapid disintegrability and solubility in the buccal cavity, and an appropriate hardness, without using special fillers, an apparatus, or the like.

EP 2 062 599 A1

**Description**

TECHNICAL FIELD

The present invention relates to a tablet rapidly disintegrating in the buccal cavity, comprising a treated starch having a degree of gelatinization of 30% to 60% which is dispersed in the tablet, and further comprising a drug and a saccharide, and a process for manufacturing the same.

BACKGROUND ART

Recently, various rapidly disintegrating tablets in the buccal cavity capable of being taken by elderly people or children without water have been developed. Such rapidly disintegrating tablets in the buccal cavity need a sufficient disruptive strength (i.e., a tablet hardness) that they are not easily disintegrated by external forces in the processes of production (including packaging), distribution, and use in the medical field. However, a disintegration rate is generally incompatible with a tablet strength, and therefore, both should be carefully balanced.

For example, Zydis (registered trademark), a medicament commercially available from Cardinal health, is manufactured by lyophilization, and thus, special manufacturing equipment such as a lyophilizer is required. Further, since the tablet strength of this medicament is low, it is difficult to push out the tablet from the pocket of a PTP (Press Through Package). This medicament having such a difficulty does not suit elderly people.

Further, some rapidly disintegrating tablets in the buccal cavity manufactured by tabletting, instead of lyophilization, have been reported. For example, patent reference 1 discloses a rapidly disintegrating tablet in the buccal cavity manufactured by kneading a mixture of a drug and additives such as a filler or a binder, together with water or the like, and compressing and drying the resulting wet paste. Patent reference 2 discloses a rapidly disintegrating tablet in the buccal cavity manufacturing by compression-molding a mixture containing a drug, a saccharide, and a barely sufficient amount of water to moisten the saccharide at a low pressure into a tablet form, and drying the resulting tablet. However, in these methods, since wet mixture should be compressed, a special tabletting machine by which adhesion thereof to a punch is avoided is required, and these methods are not generally used.

Furthermore, a rapidly disintegrating tablet in the buccal cavity containing as a main component a disintegrator has been reported. For example, patent reference 3 discloses the use of a low-substituted hydroxypropyl cellulose (L-HPC) which was specially refined, but this disintegrator is not generally used.

Starches are widely used as a general filler for solid preparations. For example, patent reference 4 discloses a method of manufacturing a tablet preparation characterized in that a starch, a water-soluble excipient, and an active ingredient are contained, the starch and the water-soluble excipient account for not less than 50% of the preparation, and the starch accounts for 5 to 50% of the starch and the water-soluble excipient. However, the tablet strength thereof is only approximately 3 to 5 kg. Patent reference 5 discloses a method of manufacturing a rapidly disintegrating tablet in the buccal cavity containing mannitol, a disintegrator, a cellulose or a derivative thereof, a lubricant, and at least one of starches and lactose. This tablet contains mannitol as a base, and substantially contains super-disintegrators such as crospovidone, croscarmellose, and the like, and the tablet hardness thereof is only approximately 40 to 50 N. Patent reference 6 discloses a rapidly disintegrating tablet in the buccal cavity consisting of a compression-molded product derived from granules obtained by granulating a sugar or a sugar alcohol which may be used as a filler for tablets, together with water-insoluble and hydrophilic granulation components such as a starch, flour containing a starch, superfine silicic anhydride, hydroxypropylstarch, and/or crospovidone. However, the substantial tablet strength is only approximately 2 to 4 kg. As described above, a sufficient hardness could not be achieved by these methods, and there is still room for improvement in the handling of the tablets.

Patent reference 7 discloses a method concerning partially-digested starch particles having functions as a binder, a disintegrator, and a lubricant, obtained by digesting a starch with an enzyme. However, this method requires a treatment based on a special enzymatic digestion, and is not generally used. Patent reference 8 discloses a method of improving the granule strength of a solid medical preparation, particularly granules, containing a drug and 10 to 90% by weight of starch, by treating the preparation with a heat treatment such as a microwave or the like, thereby performing gelatinization. However, no preparation of rapidly disintegrating tablets in the buccal cavity is disclosed in these references, and a method which requires a post-treatment of a solid preparation is complicated and impractical.

As described above, various rapidly disintegrating tablets in the buccal cavity are known, but a rapidly disintegrating tablet in the buccal cavity which can be easily manufactured without using special fillers, an apparatus, or the like, and has a good moldability and a sufficient disintegrability, is not known.

The unit N (Newton) of tablet strength has the following relationships:

```
9.807 N = 1 kp = 1 kg
```

[patent reference 1] Japanese Unexamined Patent Publication (Kokai) No. 6-218028 (corresponding to European Patent No. 590963)
[patent reference 2] Japanese Unexamined Patent Publication (Kokai) No. 5-271054 (corresponding to European Patent No. 553777)
[patent reference 3] Japanese Unexamined Patent Publication (Kokai) No. 2000-281564
[patent reference 4] International Publication No. 00/47233 [patent reference 5] Japanese Unexamined Patent Publication (Kokai) No. 2000-273039
[patent reference 6] Japanese Unexamined Patent Publication (Kokai) No. 2004-315483
[patent reference 7] Japanese Unexamined Patent Publication (Kokai) No. 2004-137230
[patent reference 8] Japanese Unexamined Patent Publication (Kokai) No. 2006-1924

DISCLOSURE OF INVENTION

PROBLEMS TO BE SOLVED BY THE INVENTION

The objects of the present invention are (1) to provide a rapidly disintegrating tablet in the buccal cavity, having a rapid disintegrability and solubility in the buccal cavity and an appropriate hardness, without using special fillers, an apparatus, or the like, and (2) to provide a process for manufacturing the rapidly disintegrating tablet in the buccal cavity, composed of general production processes and having a good industrial productivity.

It has been pointed out that known rapidly disintegrating tablets in the buccal cavity have a good disintegrability in the buccal cavity, but do not have a sufficient hardness when handled. The present inventors noticed that this problem is due to the lack of a filler having a good disintegrability and a good moldability. The present inventors have conducted intensive studies, and found that a treated starch having a degree of gelatinization within a specific range has excellent properties in both moldability and disintegrability, although an untreated starch generally shows a good disintegrability, but a poor moldability. The present inventors have further conducted intensive studies on this finding, and found that a rapidly disintegrating tablet in the buccal cavity having a high physical strength sufficient for rapidly disintegrating tablets in the buccal cavity and a good disintegrability in the buccal cavity when taken can be obtained (1) by forming a mixture containing a treated starch having a degree of gelatinization within a specific range into tablets, or (2) by granulating a mixture containing, as a general filler, a treated starch having a degree of gelatinization within a specific range, and completed the present invention. Although rapidly disintegrating tablets in the buccal cavity containing starches are known, a technique for preparing a treated starch having a specific degree of gelatinization obtained by treating a starch is not known, and it is not known that such a treated starch having a specific degree of gelatinization is useful as a filler for rapidly disintegrating tablets in the buccal cavity having a good moldability and a good disintegrability.

MEANS FOR SOLVING THE PROBLEMS

The present invention relates to:

[1] a rapidly disintegrating tablet in the buccal cavity, comprising a treated starch having a degree of gelatinization of 30% to 60%, a drug, and a saccharide, wherein the treated starch is dispersed in the tablet,
[2] the rapidly disintegrating tablet in the buccal cavity of [1], wherein the treated starch is one or two or more starches selected from the group consisting of corn starch, wheat starch, potato starch, rice starch, and cassava starch,
[3] the rapidly disintegrating tablet in the buccal cavity of [1], wherein the treated starch is corn starch,
[4] a process for manufacturing a rapidly disintegrating tablet in the buccal cavity, characterizing by comprising the steps of mixing a drug, a treated starch having a degree of gelatinization of 30% to 60%, and a saccharide, and tabletting the mixture,
[5] use of a treated starch having a degree of gelatinization of 30% to 60% as a filler and/or a binder for the manufacture of a rapidly disintegrating tablet in the buccal cavity,
[6] the use of [5], wherein the treated starch is one or two or more starches selected from the group consisting of corn starch, wheat starch, potato starch, rice starch, and cassava starch,
[7] the use of [5], wherein the treated starch is corn starch,
[8] a rapidly disintegrating tablet in the buccal cavity, comprising a treated corn starch, a drug, and lactose and/or D-mannitol,

[9] the rapidly disintegrating tablet in the buccal cavity of [8], further comprising a starch paste,

[10] the rapidly disintegrating tablet in the buccal cavity of [9], obtained by granulating the treated corn starch, the drug, and lactose and/or D-mannitol with a starch paste,

[11] the rapidly disintegrating tablet in the buccal cavity of [8] to [10], further comprising crystalline cellulose,

[12] a rapidly disintegrating tablet in the buccal cavity, comprising a treated corn starch, a drug, a saccharide having a low moldability, and a saccharide having a high moldability,

[13] a process for manufacturing a rapidly disintegrating tablet in the buccal cavity, comprising the steps of granulating a treated corn starch, a drug, and a saccharide with a starch paste, mixing the granules with a filler, and tabletting the mixture,

[14] the process for manufacturing a rapidly disintegrating tablet in the buccal cavity of [13], wherein the saccharide is lactose and/or D-mannitol, and

[15] the process for manufacturing a rapidly disintegrating tablet in the buccal cavity of [13], wherein the filler contains crystalline cellulose.

The term "rapidly disintegrating tablet in the buccal cavity" as used herein means a tablet which is disintegrated in the buccal cavity within 1 minute, preferably 40 seconds, more preferably 30 seconds, by substantially only saliva, without taking water for swallowing the tablets.

This disintegration time in the buccal cavity is a value measured under conditions in which the natural movement of the tongue or the like is not limited when a tablet is placed in the buccal cavity. For example, a tablet to be measured is placed in the buccal cavity of a healthy adult male without taking water in the mouth, and the time until the tablet is completely disintegrated and dissolved by saliva alone is measured to determine the tablet disintegration time in the buccal cavity. During the measurement, it is not necessary to limit the natural movement of the tongue or the like.

In the present invention, it was judged that the tablet had a good disintegrability in the buccal cavity when the disintegration time in the buccal cavity was within 40 seconds.

The term "gelatinization" as used herein means a swelling caused by physically treating a starch, thereby introducing water into the space between starch molecules. An increasing degree of gelatinization means the progress of gelatinization. In the present specification, the degree of gelatinization is determined by a conventional method, a glucoamylase method (Jiro NIKUNI ed., "Denpun-kagaku handbook", Asakura-Shoten, 1977, p.242), in accordance with the following procedure.

A solution is prepared by dissolving 1 g of glucoamylase in 100 mL of water, and the supernatant is used as an enzyme solution. To 100 mg (converted as dry weight) of each sample, 8 mL of water is added to prepare a suspension. Aliquots (2 mL per test tube) of the suspension are added to two test tubes. One aliquot is used to be assayed (hereinafter referred to as the liquid to be assayed), and the other is used to be completely gelatinized (hereinafter referred to as the liquid for complete gelatinization). To the liquid to be assayed, 1.6 mL of a 2 mol/L acetate buffer (pH 4.8), 0.4 mL of water, and 1 mL of the above enzyme solution are added. To the liquid for complete gelatinization, 0.2 mL of 10 N NaOH, 1.6 mL of 2 mol/L acetic acid, 0.2 mL of water, and 1 mL of the enzyme solution are added. These liquids are incubated at 37°C for 60 minutes. To 0.5 mL of each reaction liquid, 10 mL of 25 mmol/L HCl is added to stop the reaction. To 0.5 mL of the supernatant of collected from each reaction liquid, 0.5 mL of water and 1.0 mL of a Somogyi reagent are added. The mixture is heated in boiling water for 10 minutes, and cooled. Further, 1 mL of a Nelson reagent is added to the mixture. After 3 minutes from the addition, the liquid volume is adjusted with purified water to 10 mL, and the absorbance at the wavelength of 660 nm is measured. The degree of gelatinization was determined by the following equation:

$$[\text{Degree of gelatinization}] = [(Aa-Ao)/(Ab-Ao)] \times 100$$

wherein Aa is the absorbance of the liquid to be assayed, Ab is the absorbance of the liquid for complete gelatinization, and Ao is the absorbance of a blank.

The term "dispersed in a tablet" means that a treated starch having a degree of gelatinization of 30% to 60% is present over the whole area of a tablet. That is, a treated starch having a degree of gelatinization of 30% to 60% is properly mixed with other fillers or the like before tabletting, and embodiments in which only a treated starch having a degree of gelatinization beyond the range of 30% to 60% is contained as the treated starch are excluded from the present invention. For example, when tablets are prepared by mixing a treated starch having a degree of gelatinization of less than 30% with another treated starch having a degree of more than 60%, a treated starch having a degree of 30% to 60% is not present in the tablets, and the effects of the present invention are not achieved. When the tablet is a multilayer tablet such as a trilayer, or a dry coated tablet, the above term means that a treated starch having a degree of gelatinization specified in the present invention is dispersed in an area to which the disintegrability in the buccal cavity is added. That is, it is not essential that such a treated starch is dispersed in all of the layers of a multilayer tablet or in both the core tablet and the outer layer of a dry coated tablet.

The term "hardness (of a tablet)" as used herein means hardness of a tablet formed by tabletting or the like. For example, the hardness of a tablet may be indicated as a force required for crushing the tablet (unit: N). The higher the hardness, the stronger the tablet. In view of the productivity and a storage stability in distribution, rapidly disintegrating tablets in the buccal cavity were evaluated by using as a standard the value 50 N, determined by, for example, a Schleuniger tablet hardness meter (manufactured by Schleuniger Co., Ltd.) commonly used in the measurement of tablet hardness.

EFFECTS OF THE INVENTION

The rapidly disintegrating tablet in the buccal cavity of the present invention has a good disintegrability and solubility in the buccal cavity, and thus, can be easily swallowed. Further, the tablet has an appropriate strength, and thus, has a good storage stability in the production or distribution thereof. Therefore, the rapidly disintegrating tablet in the buccal cavity of the present invention can be preferably used for the treatment or prevention of patients applicable in accordance with an active ingredient contained therein, particularly elderly people or children. In addition, the tablet has a good productivity and can be provided at a low price, because the tablet can be manufactured by conventional methods using cheap materials.

BEST MODE FOR CARRYING OUT THE INVENTION

Hereinafter, the rapidly disintegrating tablet in the buccal cavity of the present invention and the process of the present invention for manufacturing the same will be explained in detail.

The drug which may be used in the present invention is not particularly limited, so long as it is an active ingredient which is therapeutically or preventively effective. As the drugs, there may be mentioned, for example, drugs for the circulatory system, such as isosorbid nitrate, nifedipine, barnidipine hydrochloride, nicardipine hydrochloride, indenolol hydrochloride, furosemide, spironolactone, guanetidine nitrate, reserpine, amosulalol hydrochloride, lisinopril, methoprolol, pilocarbpine or the like, BPH (Benign Prostatic Hyperplasia) therapeutic agents, such as tamsulosin hydrochloride or the like, antiasthmatics, such as theophylline or the like, agents for improving peripheral circulation, such as prostaglandin I derivatives such as beraprost sodium or the like, antithrombotics, hypotensives, agents for treatment of heart failure, agents for treatment of various complications of diabetes, agents for treatment of peptic ulcer, agents for treatment of skin ulcers, agents for treatment of hyperlipidemia, or the like. The drug may be used in a free form or as a pharmaceutically acceptable salt thereof. The drug may be used alone or as a combination of two or more drugs. The content of the drug is not particularly limited, so long as it is present in an amount effective for a treatment. The content of the drug is generally 80 w/w% or less, preferably 50 w/w% or less, more preferably 20 w/w% or less, with respect to the weight of a tablet. The lower limit of the content of the drug is not particularly limited, so long as it is present in an amount effective for a treatment or a prevention. The content of the drug is 0.001 w/w% or more.

The treated starch which may be used in the present invention is a starch in which the degree of gelatinization is adjusted to 30% to 60%. A method for preparing the treated starch is not particularly limited, so long as a treated starch having a specified degree of gelatinization can be prepared. For example, the treated starch may be prepared by adding water and/or energy to a starch. The energy includes thermal energy, shear energy, and the like, and is not particularly limited, so long as the gelatinization specified in the present invention can be achieved. For example, the treated starch may be prepared by adding water and/or heat to a starch. The gelatinization may be carried out, for example, by a mechanochemical treatment, such as a planetary ball mill, a vibration ball mill, a mechanofusion, or the like, or a heat treatment, such as an oven, a microwave, or the like. For example, a treated starch may be prepared by a treatment with a ball mill at 100 to 300 rpm for 1 to 36 hours, or with a mechanofusion at 4000 to 6000 revolutions for 5 to 60 minutes. Further, a starch with appropriate moisture may be allowed to stand in an oven at 50 to 100°C for 1 to 12 hours, or a starch without any pretreatment may be allowed to stand in a constant temperature and humidity chamber at 50 to 100°C under a humidity condition of 50 to 90% RH for 1 to 12 hours, to prepare a treated starch. Furthermore, a starch paste, previously obtained by adding an appropriate amount of water to a starch and heating the mixture, may be appropriately mixed with a starch prepared by another method, to prepare a treated starch. As described in Comparative Example 1 described below, the degree of gelatinization in normal corn starch is about 18%.

The origin of a starch used in the present invention is not particularly limited, and there may be mentioned, for example, corn starch, wheat starch, potato starch, rice starch, cassava starch, or the like. In particular, corn starch commonly used in Japan, the United States, and Europe is preferable in view of practical use. Such a starch may be used in the present invention by appropriately adjusting the degree of gelatinization thereof. The treated starch(es) may be used alone, or as a combination of two or more thereof.

The degree of gelatinization of the treated starch used in the present invention is 30 to 60%, preferably 30 to 50%, more preferably 40 to 50%. The treated starch may account for 5 to 90% by weight, preferably 10 to 90% by weight, more preferably 10 to 50% by weight, still more preferably 10 to 40% by weight, most preferably 20 to 40% by weight, with respect to the whole weight of the rapidly disintegrating tablet in the buccal cavity of the present invention.

Embodiments of the treated starch used in the present invention will be clarified from Examples described below, and the treated starch may be used in accordance with various methods, so long as the effects of the present invention may be achieved. For example, the treated starch adjusted to a degree of gelatinization specified in the present invention may be mixed with other components, and optionally further granulated, in a similar fashion to that using conventional fillers. Further, part or the whole of the treated starch having a degree of gelatinization specified in the present invention may be used as a binder to obtain granules. That is, the treated starch used in the present invention may be used as a filler and/or a binder for the rapidly disintegrating tablet in the buccal cavity. Further, in the use, according to the present invention, of a treated starch having a degree of gelatinization of 30 to 60% as a filler and/or a binder, the above-mentioned treated starch having a degree of gelatinization of 30 to 60% may be directly used.

The saccharide which may be used in the present invention is not particularly limited, so long as it is commonly used as a filler, and is a pharmaceutically acceptable sugar or sugar alcohol. Such a saccharide is generally dissolved in the buccal cavity, and may be preferably a saccharide having a low moldability as disclosed in WO 95/20380, for example, xylitol, erythritol, glucose, mannitol, sucrose, or lactose. The saccharide may be used alone or as a combination of two or more thereof. The term "(saccharide) having a low moldability" as used herein means that, for example, when 150 mg of saccharide is formed into tablets using a punch 8 mm in diameter under a tabletting pressure of 10 to 50 kg/cm$^2$, the tablets show a hardness of 0 to 2 kg.

In addition to the above saccharide, other fillers may be contained in the rapidly disintegrating tablet in the buccal cavity of the present invention, if desired. As the fillers, there may be mentioned, for example, various pharmaceutically acceptable additives, such as a diluent (an extender), a disintegrator, an acidulant, an effervescent agent, an artificial sweetener, a flavor, a lubricant, a coloring agent, a stabilizer, or the like. These additives may be used alone, or as a combination of two or more thereof. The content of each additive is not particularly limited, so long as it is within a pharmaceutically acceptable range.

The content of the saccharide used in the present invention may be appropriately adjusted in accordance with a dose of the drug and/or a size of the tablet. More particularly, a size of the tablet may be appropriately adjusted to a desired size, by increasing the content of fillers used in the present invention when a dose of the drug is low, or by decreasing the content of the fillers when a dose of the drug is high. The content per tablet is generally 20 to 1000 mg, preferably 50 to 500 mg, more preferably 100 to 400 mg. The percentage by weight is 30 to 99.5 w/w%, preferably 50 to 95 w/w%, with respect to the weight of the tablet.

In addition to the use of the treated starch as a binder, various binders other than the treated starch may be used in the present invention, if desired. As the binder optionally used, a starch paste, water-soluble polymers, or saccharides having a high moldability as disclosed in WO 95/20380 is preferable. The term "(saccharide) having a high moldability" as used herein means that, for example, when 150 mg of saccharide is formed into tablets using a punch 8 mm in diameter under a tabletting pressure of 10 to 50 kg/cm$^2$, the tablets show a hardness of 2 kg or more. As the water-soluble polymers, there may be mentioned, for example, hydroxypropylcellulose, hydroxypropylmethylcellulose, polyvinylpyrrolidone, polyvinyl alcohol, or the like. As the saccharide having a high moldability, there may be mentioned, for example, maltose, trehalose, sorbitol, or maltitol. The treated starch may be used alone as described above, or together with one or two or more of a starch paste, water-soluble polymers, and saccharide having a high moldability.

The content of binders other than the treated starch is 0.5 to 25 w/w%, preferably 0.5 to 20 w/w%, more preferably 0.5 to 10 w/w%, with respect to the whole weight of the treated starch and the saccharide (or, when containing other fillers, with respect to the whole weight of the treated starch, the saccharide, and the fillers), or 0.5 to 20 w/w%, preferably 2 to 20 w/w%, with respect to the whole weight of the tablet.

The diluents which may be used in the present invention include, for example, microcrystalline cellulose, saccharides such as mannitol, lactose, and the like. The disintegrators include, for example, carmellose calcium, croscarmellose sodium, low-substituted hydroxypropyl cellulose, crospovidone, and the like. The acidulants include, for example, citric acid, tartaric acid, malic acid, and the like. The effervescent agents include, for example, sodium bicarbonate and the like. The artificial sweeteners include, for example, saccharin sodium, dipotassium glycyrrhizinate, aspartame, stevia, thaumatin, and the like. The flavors include, for example, lemon, lemon-lime, orange, menthol, and the like. The lubricants include, for example, magnesium stearate, calcium stearate, sucrose fatty acid esters, polyethylene glycol, talc, stearic acid, and the like. The coloring agents include, for example, food colors such as food yellow No. 5, food red No. 2, food blue No. 2, and the like, food lake color, iron oxide, and the like. The stabilizer may be selected in accordance with a drug used. These additives may be contained alone or as a combination of two or more thereof in an appropriate amount(s).

Hereinafter, each step in the process for manufacturing a rapidly disintegrating tablet of the present invention, particularly the manufacturing conditions and the like, will be explained in detail.

Step (a): Preparation step of treated starch

In the present invention, the gelatinization of a starch may be carried out, for example, by a mechanochemical treatment, such as a planetary ball mill, a vibration ball mill, a mechanofusion, or the like, or a heat treatment, such as an oven, a microwave, or the like. Further, a starch paste, previously obtained by adding an appropriate amount of water to a starch and heating the mixture, may be appropriately mixed with a starch prepared by another method, to prepare a treated starch.

Step (b): Granulation step

A granulation method used in the present invention is not particularly limited, so long as a drug is granulated with a filler. As the granulation method, there may be mentioned, for example, a spray-drying, a fluidized bed granulation, an agitation granulation, an oscillating granulation, or the like. For example, in the fluidized bed granulation, a drug, a filler, and the treated starch may be conveyed in a fluidized bed granulator, and sprayed with a binder solution to form the whole into granules. As the binder solution, a starch paste adjusted to a concentration of approximately 1 to 10% by heating a starch suspension in water, a maltose solution or a solution of a water-soluble polymer, having a concentration of approximately 2 to 20%, or the like may be used. In the fluidized bed granulation, the volume of air, the liquid volume of the binder, the air pressure of the spray, or the like may be appropriately adjusted to maintain the temperature of the product at 25 to 40°C, and appropriate conditions may be selected in accordance with the drug.

For example, in the agitation granulation or the oscillating granulation, a drug, the treated starch having the degree of gelatinization of 30 to 60%, and other filler(s) needed may be mixed, and granulated together with a binder such as a previously gelatinized starch, a maltose solution, or a solution of a water-soluble polymer.

Further, a drug and a filler (including or excluding the treated starch) may be granulated with a binder (excluding the treated starch) by the above granulation method, and the treated starch previously adjusted may be added to the resulting granules.

The granulation is not limited to the above exemplified granulation methods, and various granulation methods capable of adjusting the gelatinization may be selected.

Step (C): Tablet forming step

The tablet forming step in the process of the present invention may be carried out in accordance with a known method, and is not limited, so long as the granules may be formed into a tablet, under not less than a minimum pressure capable of maintaining the form of the obtained tablet. The tablet forming may be carried out by using a conventional tabletting machine, for example, a single-punch tabletting machine, a rotary tabletting machine, or the like. The tabletting pressure may be generally 3 to 20 kN/punch, preferably 5 to 12 kN/punch.

EXAMPLES

The present invention now will be further illustrated by, but is by no means limited to, the following Examples. The following examples, in which a tablet hardness test, a disintegration test in the buccal cavity, and the like were carried out, include examples in which a tablet not containing a drug was evaluated. These examples may be regarded as an example of a tablet containing 0.001 W/W% of a drug, because it is considered that the items to be evaluated are little affected by the presence or absence of such a drug.

Evaluation methods

Each rapidly disintegrating tablet in the buccal cavity was evaluated in accordance with the following test methods in the following examples.

(1) Hardness test

A tablet hardness was measured using a Schleuniger tablet hardness meter (manufactured by Schleuniger Co., Ltd.). The tablet hardness is represented by a force needed to crush the tablet (unit: N). A higher value indicates a stronger tablet.

(2) Disintegration test in the buccal cavity

A tablet was placed in the buccal cavity of a healthy adult male without taking water in the mouth, and the time until the tablet was completely disintegrated and dissolved by saliva alone was measured.

(3) Gelatinization test

A conventional method, a glucoamylase method, was used in determining the degree of gelatinization.

Example 1

A treated corn starch having a degree of gelatinization of 43.7% was obtained by pulverizing 200 g of corn starch (Nihon Shokuhin Kako Co., Ltd.) using a planetary ball mill (ITOH) for 12 hours. From the resulting treated corn starch, 1 g of the treated corn starch was taken and mixed with 8.95 g of granulated lactose (Product name: Flowlac100; Meggle) and 0.05 g of magnesium stearate (Merck). The mixture was formed into tablets under a pressure of 12 kN using a tabletting machine (Shimadzu Corporation) with a punch having a diameter of 8.0 mm and a curvature of 9.6 R, to obtain tablets (200 mg/tablet). The hardness of the tablet was 55 N, and the disintegration time in the buccal cavity was 15 seconds.

Example 2

From the treated corn starch prepared in Example 1, 2 g of the treated corn starch was taken and mixed with 7.95 g of granulated lactose (Product name: Flowlac100; Meggle) and 0.05 g of magnesium stearate (Merck). The mixture was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 55 N, and the disintegration time in the buccal cavity was 16 seconds.

Example 3

From the treated corn starch prepared in Example 1, 3 g of the treated corn starch was taken and mixed with 6.95 g of granulated lactose (Product name: Flowlac100; Meggle) and 0.05 g of magnesium stearate (Merck). The mixture was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 63 N, and the disintegration time in the buccal cavity was 21 seconds.

Example 4

A treated corn starch having a degree of gelatinization of 58.1% was obtained by pulverizing 200 g of corn starch (Nihon Shokuhin Kako Co., Ltd.) using a planetary ball mill (ITOH) for 36 hours. From the resulting treated corn starch, 2 g of the treated corn starch was taken and mixed with 7.95 g of granulated lactose (Product name: Flowlac100; Meggle) and 0.05 g of magnesium stearate (Merck). The mixture was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 57 N, and the disintegration time in the buccal cavity was 35 seconds.

Example 5

A treated corn starch having a degree of gelatinization of 50.5% was obtained by pulverizing 200 g of corn starch (Nihon Shokuhin Kako Co., Ltd.) using a planetary ball mill (ITOH) for 24 hours. From the resulting treated corn starch, 2 g of the treated corn starch was taken and mixed with 7.95 g of lactose (DMV) and 0.05 g of magnesium stearate (Merck). The mixture was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 72 N, and the disintegration time in the buccal cavity was 26 seconds.

Example 6

A starch paste was prepared by heating 4.0 g of corn starch (Nihon Shokuhin Kako Co., Ltd.) in 36.0 g of purified water. Into a vertical mixer (Shinagawa Machinery Works Co., Ltd.), 198.8 g of lactose (DMV), 46.0 g of the treated corn starch prepared in Example 1, and 40.0 g of the resulting starch paste, were loaded and granulated. The granules were sifted through a 20 mesh screen, and dried at 40°C overnight. From the resulting granulated product, 9.95 g of the granulated produce was taken and mixed with 0.05 g of magnesium stearate (Merck). The mixture was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 79 N, and the disintegration time in the buccal cavity was 32 seconds.

Example 7

Into a vertical mixer (Shinagawa Machinery Works Co., Ltd.), 198.8 g of D-mannitol (Roquette), 46.0 g of the treated corn starch prepared in Example 1, and 40.0 g of the starch paste prepared in Example 6 were loaded and granulated. The granules were sifted through a 20 mesh screen, and dried at 40°C overnight. From the resulting granulated product, 9.95 g of the granulated produce was taken and mixed with 0.05 g of magnesium stearate (Merck). The mixture was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 66 N, and the disintegration time in the buccal cavity was 29 seconds.

Example 8

Into a fluidized bed granulator (Glatt), 447 g of lactose (DMV) and 120.0 g of the treated corn starch prepared in Example 1 were loaded, and granulated with a maltose aqueous solution which had been prepared by dissolving 30 g of maltose (Sunmalt S; Hayashibara Shoji, Inc.) in 170 g of purified water. The granules were dried, and sifted through a 20 mesh screen. From the resulting granulated product, 398 g of the granulated produce was taken and mixed with 2 g of magnesium stearate (Merck). The mixture was formed into tablets under a pressure of 8 kN using a rotary tabletting machine (X20; Hata Iron Works Co., Ltd.) with a punch having a diameter of 8.0 mm and a curvature of 9.6 R, to obtain tablets (200 mg/tablet). The hardness of the tablet was 95 N, and the disintegration time in the buccal cavity was 25 seconds.

Example 9

Into a fluidized bed granulator (Glatt), 447 g of D-mannitol (Roquette) and 120.0 g of the treated corn starch prepared in Example 1 were loaded, and granulated with a maltose aqueous solution which had been prepared by dissolving 30 g of maltose (Sunmalt S; Hayashibara Shoji, Inc.) in 170 g of purified water. The granules were dried, and sifted through a 20 mesh screen. From the resulting granulated product, 398 g of the granulated produce was taken and mixed with 2 g of magnesium stearate (Merck). The mixture was formed into tablets under a pressure of 8 kN using a rotary tabletting machine (X20; Hata Iron Works Co., Ltd.) with a punch having a diameter of 8.0 mm and a curvature of 9.6 R, to obtain tablets (200 mg/tablet). The hardness of the tablet was 88 N, and the disintegration time in the buccal cavity was 30 seconds.

Example 10

Into an agitation granulator (Powrex Corp.), 397.5 g of D-mannitol (Roquette), 92.5 g of the treated corn starch prepared in Example 1, and 75.0 g of the starch paste prepared in Example 6 were loaded and granulated. The granules were dried in a fluidized bed granulator (Glatt), and sifted through a 20 mesh screen. From the resulting granulated product, 398 g of the granulated produce was taken and mixed with 2 g of magnesium stearate (Merck). The mixture was formed into tablets under a pressure of 10 kN using a rotary tabletting machine (X20; Hata Iron Works Co., Ltd.) with a punch having a diameter of 8.0 mm and a curvature of 9.6 R, to obtain tablets (200 mg/tablet). The hardness of the tablet was 59 N, and the disintegration time in the buccal cavity was 35 seconds.

Example 11

Into an agitation granulator (Powrex Corp.), 397.5 g of lactose (DMV), 92.5 g of the treated corn starch prepared in Example 1, and 75.0 g of the starch paste prepared in Example 6, were loaded and granulated. The granules were dried in a fluidized bed granulator (Glatt), and sifted through a 20 mesh screen. From the resulting granulated product, 398 g of the granulated produce was taken and mixed with 2 g of magnesium stearate (Merck). The mixture was formed into tablets under a pressure of 10 kN using a rotary tabletting machine (X20; Hata Iron Works Co., Ltd.) with a punch having a diameter of 8.0 mm and a curvature of 9.6 R, to obtain tablets (200 mg/tablet). The hardness of the tablet was 56 N, and the disintegration time in the buccal cavity was 35 seconds.

Example 12

A treated corn starch having a degree of gelatinization of 56.2% was obtained by heating 1200 g of corn starch (Nihon Shokuhin Kako Co., Ltd.) at 300°C using a Meteo Rainbow (Nippon Pneumatic Mfg. Co., Ltd.). From the resulting treated corn starch, 2 g of the treated corn starch was taken and mixed with 7.95 g of D-mannitol (Roquette) and 0.05 g of magnesium stearate (Merck). The mixture was formed into tablets under a pressure of 12 kN using a tabletting machine (Shimadzu Corporation) with a punch having a diameter of 8.0 mm and a curvature of 9.6 R, to obtain tablets (200 mg/tablet). The hardness of the tablet was 52 N, and the disintegration time in the buccal cavity was 20 seconds.

Example 13

A treated corn starch having a degree of gelatinization of 38.8% was obtained by heating 100 g of corn starch (Nihon Shokuhin Kako Co., Ltd.) at 70°C and 80% RH in a Temperature and Humidity Chamber (Espec Corp.). From the resulting treated corn starch, 2 g of the treated corn starch was taken and mixed with 7.95 g of granulated lactose (Product name: Flowlac100; Meggle) and 0.05 g of magnesium stearate (Merck). The mixture was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 51 N, and the disintegration time in the buccal cavity was 15 seconds.

Example 14

A treated corn starch having a degree of gelatinization of 34.8% was obtained by pulverizing 70 g of corn starch (Nihon Shokuhin Kako Co., Ltd.) using a mechanofusion (Hosokawa Micron Corporation) at 5500 revolutions for 30 minutes. From the resulting treated corn starch, 2 g of the treated corn starch was taken and mixed with 7.95 g of D-mannitol (Roquette) and 0.05 g of magnesium stearate (Merck). The mixture was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 58 N, and the disintegration time in the buccal cavity was 15 seconds.

Example 15

A mixture was obtained by mixing 1 g of famotidine, 2 g of the treated corn starch prepared in Example 1, 6.95 g of D-mannitol (Roquette), and 0.05 g of magnesium stearate (Merck), and was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 64 N, and the disintegration time in the buccal cavity was 25 seconds.

Example 16

A mixture was obtained by mixing 1 g of acetaminophen, 2 g of the treated corn starch prepared in Example 1, 6.95 g of D-mannitol (Roquette), and 0.05 g of magnesium stearate (Merck), and was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 51 N, and the disintegration time in the buccal cavity was 20 seconds.

Example 17

Into a fluidized bed granulator (Glatt), 185 g of D-mannitol (Roquette) and 50 g of the treated corn starch prepared in Example 14 were loaded, and granulated with a starch paste which had been prepared by heating 1.25 g of corn starch (Nihon Shokuhin Kako Co., Ltd.) in 123.75 g of purified water. The granules were dried and sifted through a 20 mesh screen. From the resulting granulated product, 212.7 g of the granulated produce was taken and mixed with 11.3 g of crystalline cellulose (Ceolus PH102; Asahi Kasei Chemicals Corporation) and 1.13 g of magnesium stearate (Merck). The mixture was formed into tablets under a pressure of 8 kN using a rotary tabletting machine (EX10; Hata Iron Works Co., Ltd.) with a punch having a diameter of 8.0 mm and a curvature of 12 R, to obtain tablets (200 mg/tablet). The hardness of the tablet was 52 N, and the disintegration time in the buccal cavity was 15 seconds.

Example 18

A treated corn starch having a degree of gelatinization of 37.2% was obtained by pulverizing 70 g of corn starch (Nihon Shokuhin Kako Co., Ltd.) using a mechanofusion (Hosokawa Micron Corporation) at 5000 revolutions for 60 minutes. Into a fluidized bed granulator (Glatt), 60 g of the treated corn starch, 30 g of famotidine, and 192 g of D-mannitol (Roquette) were loaded, and granulated with a starch paste which had been prepared by heating 1.5 g of corn starch (Nihon Shokuhin Kako Co., Ltd.) in 148.5 g of purified water. The granules were dried and sifted through a 20 mesh screen. From the resulting granulated product, 254.8 g of the granulated produce was taken and mixed with 13.5 g of crystalline cellulose (Ceolus PH102; Asahi Kasei Chemicals Corporation) and 1.35 g of magnesium stearate (Merck). The mixture was formed into tablets under a pressure of 6 kN using a rotary tabletting machine (EX10; Hata Iron Works Co., Ltd.) with a punch having a diameter of 8.0 mm and a curvature of 12 R, to obtain tablets (200 mg/tablet). The hardness of the tablet was 53 N, and the disintegration time in the buccal cavity was 20 seconds. It was found that the content of the corn starch treated by mechanofusion was preferably 10 to 30% with respect to the whole weight of a preparation.

Example 19

A treated corn starch having a degree of gelatinization of 42.0% was obtained by pulverizing 70 g of corn starch (Nihon Shokuhin Kako Co., Ltd.) using a mechanofusion (Hosokawa Micron Corporation) at 5000 revolutions for 15 minutes. A mixture was obtained by mixing 2 g of famotidine, 0.4 g of the treated corn starch, 1.38 g of D-mannitol (Roquette), 0.2 g of crystalline cellulose (Ceolus PH102; Asahi Kasei Chemicals Corporation), and 0.02 g of magnesium stearate (Merck), and was formed into tablets under a pressure of 8 kN using a tabletting machine (Shimadzu Corporation) with a punch having a diameter of 8.0 mm and a curvature of 9.6 R, to obtain tablets (200 mg/tablet). The hardness of the tablet was 59 N, and the disintegration time in the buccal cavity was 20 seconds. It was found that the degree of gelatinization of the corn starch treated by mechanofusion was preferably 35 to 45%.

Comparative Example 1

The degree of gelatinization of a corn starch (Nihon Shokuhin Kako Co., Ltd.) was measured to obtain a value of 18.0%. A mixture was obtained by mixing 2 g of this corn starch, 7.95 g of granulated lactose (Product name: Flowlac100; Meggle), and 0.05 g of magnesium stearate (Merck), and was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 20 N, and the disintegration time in the buccal cavity was 17 seconds. It was found that a sufficient hardness could not be achieved when the degree of gelatinization of the starch was low.

Comparative Example 2

A treated corn starch having a degree of gelatinization of 63.8% was obtained by pulverizing 200 g of corn starch (Nihon Shokuhin Kako Co., Ltd.) using a planetary ball mill (ITOH) for 48 hours. From the resulting treated corn starch, 2 g of the treated corn starch was taken and mixed with 7.95 g of lactose (DMV) and 0.05 g of magnesium stearate (Merck). The mixture was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 51 N, and the disintegration time in the buccal cavity was 46 seconds. It was found that a good disintegrability in the buccal cavity could not be achieved when the degree of gelatinization of the starch was high.

Comparative Example 3

The degree of gelatinization of PCS (Asahi Kasei Chemicals Corporation) was measured to obtain a value of 75.9%. A mixture was obtained by mixing 2 g of the PCS, 7.95 g of granulated lactose (Product name: Flowlac100; Meggle), and 0.05 g of magnesium stearate (Merck), and was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 19 N, and the disintegration time in the buccal cavity was 80 seconds. It was found that a good disintegrability in the buccal cavity could not be achieved when the degree of gelatinization of the starch was high.

Comparative Example 4

A mixture was obtained by mixing 1 g of the corn starch used in Comparative Example 1, 1 g of the PCS used in Comparative Example 3, 7.95 g of granulated lactose (Product name: Flowlac100; Meggle), and 0.05 g of magnesium stearate (Merck), and was formed into tablets under a pressure of 10 kN using a tabletting machine (Shimadzu Corporation) with a punch having a diameter of 8.0 mm and a curvature of 9.6 R, to obtain tablets (200 mg/tablet). The hardness of the tablet was 30 N, and the disintegration time in the buccal cavity was 50 seconds.

INDUSTRIAL APPLICABILITY

The rapidly disintegrating tablet in the buccal cavity of the present invention can be preferably used in the treatment or prevention of diseases, in particular, for elderly people or children.
Although the present invention has been described with reference to specific embodiments, various changes and modifications obvious to those skilled in the art are possible without departing from the scope of the appended claims.

**Claims**

1. A rapidly disintegrating tablet in the buccal cavity, comprising a treated starch having a degree of gelatinization of 30% to 60%, a drug, and a saccharide, wherein the treated starch is dispersed in the tablet.

2. The rapidly disintegrating tablet in the buccal cavity according to claim 1, wherein the treated starch is one or two or more starches selected from the group consisting of corn starch, wheat starch, potato starch, rice starch, and cassava starch.

3. The rapidly disintegrating tablet in the buccal cavity according to claim 1, wherein the treated starch is corn starch.

4. A process for manufacturing a rapidly disintegrating tablet in the buccal cavity, **characterizing by** comprising the steps of mixing a drug, a treated starch having a degree of gelatinization of 30% to 60%, and a saccharide, and tabletting the mixture.

5. Use of a treated starch having a degree of gelatinization of 30% to 60% as a filler and/or a binder for the manufacture of a rapidly disintegrating tablet in the buccal cavity.

6. The use according to claim 5, wherein the treated starch is one or two or more starches selected from the group consisting of corn starch, wheat starch, potato starch, rice starch, and cassava starch.

7. The use according to claim 5, wherein the treated starch is corn starch.

8. A rapidly disintegrating tablet in the buccal cavity, comprising a treated corn starch, a drug, and lactose and/or D-mannitol.

9. The rapidly disintegrating tablet in the buccal cavity according to claim 8, further comprising a starch paste.

10. The rapidly disintegrating tablet in the buccal cavity according to claim 9, obtained by granulating the treated corn starch, the drug, and lactose and/or D-mannitol with a starch paste.

11. The rapidly disintegrating tablet in the buccal cavity according to any one of claims 8 to 10, further comprising crystalline cellulose.

12. A rapidly disintegrating tablet in the buccal cavity, comprising a treated corn starch, a drug, a saccharide having a low moldability, and a saccharide having a high moldability.

13. A process for manufacturing a rapidly disintegrating tablet in the buccal cavity, comprising the steps of granulating a treated corn starch, a drug, and a saccharide with a starch paste, mixing the granules with a filler, and tabletting the mixture.

14. The process according to claim 13, wherein the saccharide is lactose and/or D-mannitol.

**15.** The process according to claim 13, wherein the filler contains crystalline cellulose.

## INTERNATIONAL SEARCH REPORT

| | International application No. |
| --- | --- |
| | PCT/JP2007/067805 |

**A. CLASSIFICATION OF SUBJECT MATTER**

*A61K47/36*(2006.01)i, *A61K9/20*(2006.01)i, *A61K47/10*(2006.01)i, *A61K47/26* (2006.01)i, *A61K47/38*(2006.01)i

According to International Patent Classification (IPC) or to both national classification and IPC

**B. FIELDS SEARCHED**

Minimum documentation searched (classification system followed by classification symbols)
A61K47/36, A61K9/20, A61K47/10, A61K47/26, A61K47/38

Documentation searched other than minimum documentation to the extent that such documents are included in the fields searched
Jitsuyo Shinan Koho          1922-1996   Jitsuyo Shinan Toroku Koho   1996-2007
Kokai Jitsuyo Shinan Koho    1971-2007   Toroku Jitsuyo Shinan Koho   1994-2007

Electronic data base consulted during the international search (name of data base and, where practicable, search terms used)
BIOSIS(STN), CAplus(STN), EMBASE(STN), MEDLINE(STN), JMEDPlus(JDream2), JST7580(JDream2), JSTPlus(JDream2)

**C. DOCUMENTS CONSIDERED TO BE RELEVANT**

| Category* | Citation of document, with indication, where appropriate, of the relevant passages | Relevant to claim No. |
| --- | --- | --- |
| X<br>Y | WO 2006/085497 A1 (KISSEI PHARM CO., LTD., JP),<br>17 August, 2006 (17.08.06),<br>Full text; particularly, Claims; examples<br>(Family: none) | 1-8<br>9-15 |
| X<br>Y | JP 2000-273039 A (TAISHO PHARM CO., LTD.),<br>03 October, 2000 (03.10.00),<br>Full text; particularly, Claims; Par. No.<br>[0008]; examples 2, 6, 7<br>(Family: none) | 1-8<br>9-15 |

☒ Further documents are listed in the continuation of Box C.      ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>    22 November, 2007 (22.11.07) | Date of mailing of the international search report<br>    04 December, 2007 (04.12.07) |
| --- | --- |
| Name and mailing address of the ISA/<br>    Japanese Patent Office | Authorized officer |
| Facsimile No. | Telephone No. |

Form PCT/ISA/210 (second sheet) (April 2007)

**INTERNATIONAL SEARCH REPORT**

| International application No. |
| --- |
| PCT/JP2007/067805 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | Tadashi MAKINO, "Kokei Seizaiyo Tenkazai to Shite no α-ka Denpun ni Kansuru Kisoteki Oyobi Oyoteki Kenkyu", Journal of the Society of Powder Technology, Japan, 1996, Vol.33, No.11, pages 878 to 881, full text, particularly, page 878, right column to page 879, upper left column | 1-15 |
| Y | JP 2002-012559 A (NAT STARCH & CHEM INVESTMENT HOLDING CORP.), 15 January, 2002 (15.01.02), Full text; particularly, Par. No. [0006] & EP 1152026 A1 & US 2002/054905 A1 & US 6572887 B2 & EP 1152026 B1 | 1-15 |
| Y | US 3622677 A (STALEY MFG CO. A E), 23 November, 1971 (23.11.71), Full text; particularly, Claims; examples (Family: none) | 1-15 |
| Y | US 4072535 A (STALEY MFG CO A E), 07 February, 1978 (07.02.78), Full text; particularly, Claims; examples (Family: none) | 1-15 |
| Y | JP 58-032828 A (AJINOMOTO KABUSHIKI KAISHA), 25 February, 1983 (25.02.83), Full text; particularly, page 1, lower right column (Family: none) | 1-15 |
| Y | JP 49-110819 A (TAKEDA CHEM IND LTD.), 22 October, 1974 (22.10.74), Full text; particularly, Claims; examples & JP 78005725 B | 1-15 |
| Y | JP 04-318001 A (ASAHI CHEM IND CO., LTD.), 09 November, 1992 (09.11.92), Full text; particularly, Claims; examples & JP 3004758 B2 | 1-15 |
| Y | JP 59-047600 B2 (ASAHI KASEI KOGYO KABUSHIKI KAISHA), 20 November, 1984 (20.11.84), Full text; particularly, Claims; examples & US 4383111 A & US 4447601 A & JP 57-005700 A | 1-15 |
| Y | WO 2000/47233 A1 (SUMITOMO PHARM CO., LTD.), 17 August, 2000 (17.08.00), Full text; particularly, Claims; examples & EP 1153616 A1 & JP 2000-598184 A & US 2003/026835 A1 & EP 1153616 B1 | 1-15 |

Form PCT/ISA/210 (continuation of second sheet) (April 2007)

**INTERNATIONAL SEARCH REPORT**

| International application No. |
|---|
| PCT/JP2007/067805 |

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2004-315483 A  (TOWA YAKUHIN KABUSHIKI KAISHA),<br>11 November, 2004 (11.11.04),<br>Full text; particularly, Claims; examples<br>(Family: none) | 1-15 |
| Y | WO 95/20380 A1  (YAMANOUCHI PHARM CO., LTD.),<br>03 August, 1995 (03.08.95),<br>Full text; particularly, Claims; description,<br>page 6, the last line to page 7, line 4;<br>examples<br>& JP 07-519980 A       & US 5576014 A<br>& EP 745382 A1        & EP 745382 A4<br>& JP 3122141 B2       & EP 745382 B1 | 1-15 |

Form PCT/ISA/210 (continuation of second sheet) (April 2007)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6218028 A
- EP 590963 A
- JP 5271054 A
- EP 553777 A
- JP 2000281564 A
- JP 0047233 A
- JP 2000273039 A
- JP 2004315483 A
- JP 2004137230 A
- JP 2006001924 A
- WO 9520380 A